# EUROPEAN PATENT APPLICATION

(11) **EP 2 178 014 A1**
(43) Date of publication of application: **21.04.2010**
(21) Application number: 09171638.1
(22) Date of filing: 29.09.2009
(51) Int. Cl.: G06F 19/00

(54) **Method and apparatus for managing patients**

(30) Priority: 08.10.2008 KR 20080098781
(71) Applicant: Samsung Electronics Co., Ltd., Suwon-si, Gyeonggi-do 442-742 (KR)
(72) Inventor: Jang, Woo-young, Gyeonggi-do (KR); Nam, Yun-sun, Gyeonggi-do (KR); Yoo, Kyu-tae, Yongin-si, Gyeonggi-do (KR)
(74) Representative: Grootscholten, Johannes A.M.

(57) **Abstract**

A patient management method includes; collecting initial information about a patient, monitoring the collected initial information to determine whether the collected initial information corresponds to an event indicating an abnormal condition of the patient, selectively collecting additional information about the patient based on a result of the monitoring, and performing medical processing with regard to the patient based on at least one of the collected initial information and the additional information.

## Description

### BACKGROUND

### 1. Field

One or more exemplary embodiments relate to a patient management method and apparatus.

### 2. Description of the Related Art

In 2008, the patient management market in the United States of America ("US") amounted to 30 billion dollars and there were 78 million chronic disease patients in the US, which is 35% of the entire population. In particular, since the population of the US is aging, the patient management market will increase further in the future. The same goes for other developed countries as well as developing countries. Representative chronic diseases include diabetes, hypertension, cardiovascular disease, pneumonic disease, and other similar ailments. Patients with these chronic diseases need continuous monitoring. In this regard, since the patients need continuous care, the patients may prefer to receive medical care at home rather than in a hospital, in view of medical expenses. One method of receiving the medical care at home is to use home tele-monitoring. Research and development in this regard are being conducted.

### SUMMARY

One or more exemplary embodiments include a method and apparatus for reducing a load on a patient management system while simultaneously minimizing a workload on medical personnel and reducing patient inconvenience.

One or more exemplary embodiments include a computer readable recording medium having embodied thereon a computer program for executing the method.

Additional aspects will be set forth in part in the description which follows and, in part, will be apparent from the description, or may be learned by practice of the invention.

An exemplary embodiment of a patient management apparatus includes; a first collection unit which collects initial information about a patient, a monitor which determines whether the collected information corresponds to an event indicating an abnormal condition of the patient, a second collection unit which selectively collects additional information about the patient based on a result of the monitoring, and a processing unit which performs medical processing with regard to the patient based on at least one of the collected initial information and the additional information.

An exemplary embodiment of a patient management method includes; collecting initial information about a patient, monitoring the collected initial information to determine whether the collected initial information corresponds to an event indicating an abnormal condition of the patient, selectively collecting additional information about the patient based on a result of the monitoring, and performing medical processing with regard to the patient based on at least one of the collected initial information and the additional information.

One or more exemplary embodiments may include a computer readable recording medium having embodied thereon a computer program for executing the method described above.

### BRIEF DESCRIPTION OF THE DRAWINGS

These and/or other aspects, advantages and features will become more apparent and more readily appreciated from the following description of the exemplary embodiments, taken in conjunction with the accompanying drawings of which:
FIG. 1 is a block diagram of an exemplary embodiment of a patient management system according to the inventive concept;
FIG. 2 illustrates an exemplary embodiment of content stored in databases of a patient management gateway shown in FIG. 1 according to the inventive concept;
FIG. 3 is a detailed block diagram of an exemplary embodiment of a processing unit of a patient management gateway shown in FIG. 1 according to the inventive concept;
FIG. 4 illustrates an exemplary embodiment of a patient's blood glucose level applied to the patient management system shown in FIG. 1 according to the inventive concept; and
FIG. 5 is a flowchart illustrating an exemplary embodiment of a patient management method according to the inventive concept.

### DETAILED DESCRIPTION

The invention now will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

It will be understood that when an element is referred to as being "on" another element, it can be directly on the other element or intervening elements may be present there between. In contrast, when an element is referred to as being "directly on" another element, there are no intervening elements present. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another elements as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower", can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments of the present invention are described herein with reference to cross section illustrations that are schematic illustrations of idealized embodiments of the present invention. As such, variations from the shapes of the illustrations as a result, for example, of manufacturing techniques and/or tolerances, are to be expected. Thus, embodiments of the present invention should not be construed as limited to the particular shapes of regions illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present invention.

Hereinafter, the present invention will be described in detail with reference to the accompanying drawings.

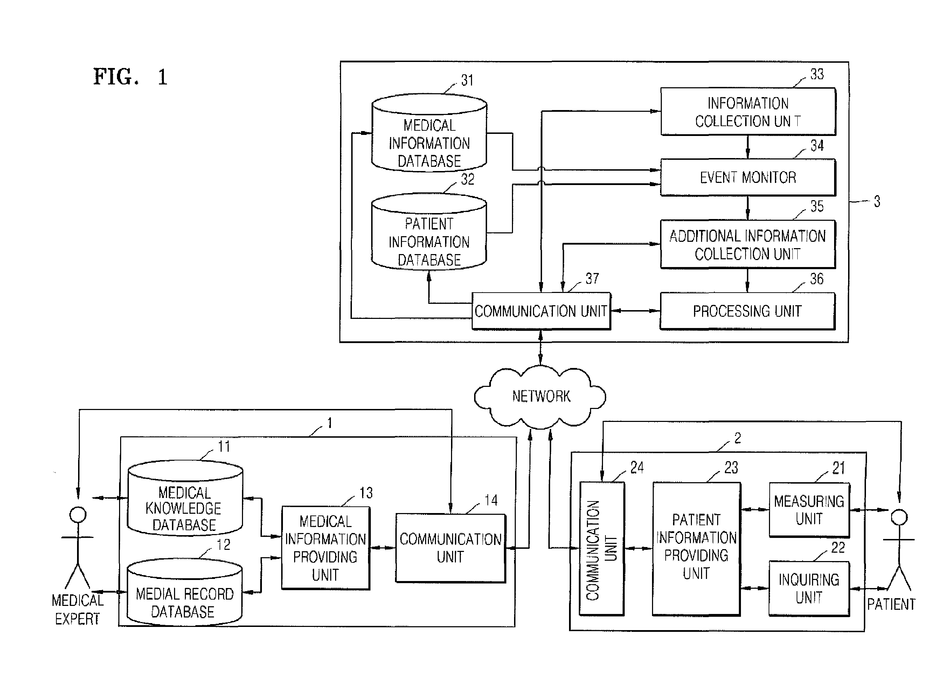

FIG. 1 is a block diagram of an exemplary embodiment of a patient management system according to the inventive concept. Referring to FIG. 1, the patient management system comprises a patient management server 1, a patient terminal 2, and a patient management gateway 3. The patient management server 1, the patient terminal 2, and the patient management gateway 3 communicate with each other, e.g., over a network, and respectively comprise communication units 14, 24, and 37 that support network communication. Although the network may be the Internet in the present exemplary embodiment, it will be understood by one of ordinary skill in the art that a different type of network, such as a wireless LAN, may be used as the network. Although the patient management server 1, the patient terminal 2, and the patient management gateway 3 are separated from each other in the present embodiment, it will be understood by one of ordinary skill in the art that the patient management server 1, the patient terminal 2, and the patient management gateway 3 may be united into a single device or a combination of two devices. For example, in one exemplary embodiment a function of the patient management gateway 3 may be embedded in the patient terminal 2.

The patient management server 1 comprises a medical knowledge database 11, a medial record database 12, a medical information providing unit 13, and the communication unit 14. The medical knowledge database 11 stores medical knowledge such as information about diseases, information about treatment of the diseases and other similar information. In particular, the medical knowledge database 11 stores information about blood glucose levels, blood pressure, pulse, temperature, and other biometric information. For example, in one exemplary embodiment the medical knowledge database 11 stores information about an abnormal range of blood glucose levels according to a person's age, height, weight, sex, and other personal characteristics. The medial record database 12 stores a patient's personal medical record. For example, in one exemplary embodiment the medial record database 12 records a diabetic patient's treatment history.

The medical information providing unit 13 transmits the medical knowledge stored in the medical information database 11 and the medical record stored in the medical record database 12 to the patient management gateway 3 through the communication unit 14. In more detail, the medical information providing unit 13 may transmit the medical knowledge stored in the medical information database 11 and the medical record stored in the medical record database 12 to the patient management gateway 3 through the communication unit 14 periodically or according to instructions given by medical personnel, e.g., a medical expert, who interacts with the patient management server 1. In the present exemplary embodiment, the medical personnel may be a doctor.

The patient terminal 2 comprises a measuring unit 21, an inquiring unit 22, a patient information providing unit 23, and the communication unit 24. The measuring unit 21 measures a bio-signal, e.g., the blood glucose level, of a patient who uses the patient terminal 2. Exemplary embodiments include configurations wherein a device for measuring the bio-signal of the patient may be included in the patient terminal 2, may be attached to the patient terminal 2 as a separate device, or may be connected to the patient terminal 2 that is spaced apart therefrom. Also, it will be understood by one of ordinary skill in the art that a different type of device may be realized. In more detail, the measuring unit 21 may measure the bio-signal of the patient that uses the patient terminal 2 periodically or according to instructions given by the patient or the medical personnel who handle the patient management gateway 3.

The inquiring unit 22 displays an inquiry regarding a patient's condition for the patient who uses the patient terminal 2. Examples of the inquiry are a personal medical profile including a physical character, a family history, a life style, medical data, and the like, tele-monitoring information including a patient's diet, ability to follow a diet, ability to consume different types of food, and capacity for diet, type and time of exercise, and other health related questions. Thereafter, the inquiring unit 22 receives a response from the patient who recognizes the inquiry. In more detail, the inquiring unit 22 may display the inquiry regarding the patient's condition periodically or according to instructions given by the patient or the medical personnel who handle the patient management gateway 3 through the communication unit 24.

The patient information providing unit 23 transmits the information about the bio-signal of the patient obtained by the measuring unit 21 and the inquiry input into the inquiring unit 22 to the patient management gateway 3 through the communication unit 24. In more detail, the patient information providing unit 23 transmits the information about the bio-signal of the patient obtained by the measuring unit 21 and the inquiry input into the inquiring unit 22 to the patient management gateway 3 periodically or according to instructions given by the patient or the medical personnel who handle the patient management gateway 3 through the communication unit 24.

In the present exemplary embodiment, the patient management gateway 3 comprises a medical information database 31, a patient information database 32, an information collection unit 33, an event monitor 34, an additional information collection unit 35, a processing unit 36, and the communication unit 37.

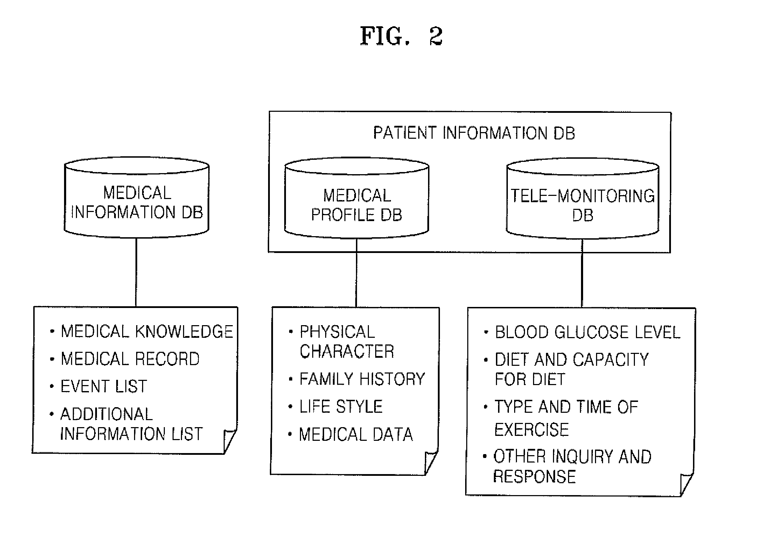

FIG. 2 illustrates exemplary embodiments of content stored in the databases of the patient management gateway 3 shown in FIG. 1. Referring to FIG. 2, the medical information database 31 stores the medical knowledge and the medical record received from the patient management server 1 through the communication unit 37. The medical information database 31 stores a list of events requiring monitoring and a list of additional information required from a patient, in addition to the medical knowledge and the medical record received from the patient management server 1. The list of events requiring monitoring and the list of additional pieces of information may be updated by the patient or the medical personnel who handle the patient management gateway 3 at any time.

The patient information database 32 comprises a medical profile database and a tele-monitoring database.
The medical profile database records a personal medical profile including a physical characteristic, a family history, a life style, medical data, and other similar information. The personal physical characteristic may be age, sex, race, height, weight, and other similar information. The family history may concern whether parents or siblings have suffered from diabetes, heart disease, hypertension, or other ailments. The life style may refer to smoking, drinking habits, exercise habits, or other aspects of a patient's life style. The medical data may be test results in relation to glycosylated hemoglobin, a blood pressure, a lipid characteristic, or other similar information.

The tele-monitoring database records tele-monitoring information received from the patient terminal 2 through the communication unit 37, e.g., information about the bio-signal of the patient measured by the device for measuring the bio-signal of the patient that is attached or connected to the patient terminal 2, the content of the inquiry regarding the patient through the patient terminal 2, and other information. An example of the information about the signal of the bio-signal of the patient may be a blood pressure level. Examples of the content of the inquiry regarding the patient may be diet in terms of types of food consumed and quantity, type and time of an exercise, a response to the inquiry, and other information.

The information collection unit 33 collects information about the patient who uses the patient terminal 2 from the patient terminal 2 through the communication unit 24. For example, in one exemplary embodiment the information collection unit 33 may collect the information about the patient periodically or when the information collection unit 33 receives patient's or medical personnel's instructions from the patient terminal 2 or the patient management server 1 through the communication unit 24 or through the communication unit 14, respectively.

The event monitor 34 monitors if the information collected by the information collection unit 33 corresponds to an event indicating an abnormal condition of a patient, based on the data stored in the medical information database 31 and the patient information database 32. The abnormal event may be a blood glucose level exceeding a patient's normal condition. In more detail, the event monitor 34 monitors if the information collected by the information collection unit 33 corresponds to any one of a plurality of events included in the list stored in the medical information database 31 based on the medical knowledge and the medical record stored in the medical information database 31, the personal medical profile of the patient stored in the patient information database 32, and the tele-monitoring information about the patient.

With regard to the blood glucose level, for example, the event monitor 34 extracts information about the blood glucose level according to a patient's age, height, and weight, which are stored in the patient information database 32, from blood glucose levels used to predict a disease stored in the medical information database 31, and, if the blood glucose level of the patient collected by the information collection unit 33 exceeds a range indicating a patient's normal condition, the event monitor 34 monitors if the extracted information corresponds to any one of a plurality of events included in the list stored in the medical information database 31.

The additional information collection unit 35 collects additional information about the patient if the information collected by the information collection unit 33 corresponds to the event indicating the abnormal condition of the patient as a result of monitoring obtained by the event monitor 34. The information collection unit 33 collects basic information about the patient, for example, in one exemplary embodiment the information collection unit 33 collects a patient's current blood glucose level, whereas the additional information collection unit 35 collects information about matters that may cause the patient's current blood glucose level, for example, diet in terms of types of food consumed and quantity, and type and time of an exercise.

In more detail, the additional information collection unit 35 requests the patient terminal 2 for the additional information about the patient through the communication unit 37 by transmitting a list of the additional information stored in the medical information database 31 to the patient terminal 2 through the communication unit 37, and obtains the additional information about the patient in response to the request by receiving the additional information about the patient from the patient terminal 2 through the communication unit 24. In this regard, the list of the additional information may relate to the blood glucose level among a plurality of lists stored in the medical information database 31. For example, in one exemplary embodiment the additional information collection unit 35 may request the additional information about the patient by transmitting the inquiry regarding the patient to the patient terminal 2 and by transmitting a message requesting to measure a specific bio-signal of the patient to the patient terminal 2.

The processing unit 36 performs medical processing with regard to the patient based on at least one of the information collected by the information collection unit 33 and the additional information collected by the additional information collection unit 35. In one exemplary embodiment, the processing unit 36 performs medical processing with regard to the patient based on both the information collected by the information collection unit 33 and the additional information collected by the additional information collection unit 35. If the information collected by the information collection unit 33 does not correspond to the event indicating an abnormal condition of the patient, since the additional information collection unit 35 does not collect the additional information about the patient, the processing unit 36 performs medical processing with regard to the patient based on the information initially collected by the information collection unit 33. If the information initially collected by the information collection unit 33 corresponds to the event indicating an abnormal condition of the patient, the processing unit 36 performs medical processing with regard to the patient based on the additional information collected by the additional information collection unit 35. However, the processing unit 36 may perform medical processing with regard to the patient based on the information collected by the information collection unit 33 and the additional information collected by the additional information collection unit 35 as described above.

In more detail, the processing unit 36 selects any one of a plurality of medical processing methods based on at least one of the information initially collected by the information collection unit 33 and the additional information collected by the additional information collection unit 35, and performs medical processing with regard to the patient according to the selected medical processing method. In the present exemplary embodiment, the medical processing methods include a manual processing method that requires decision making of the medical personnel and an automatic processing method that requires no decision making by the medical personnel.

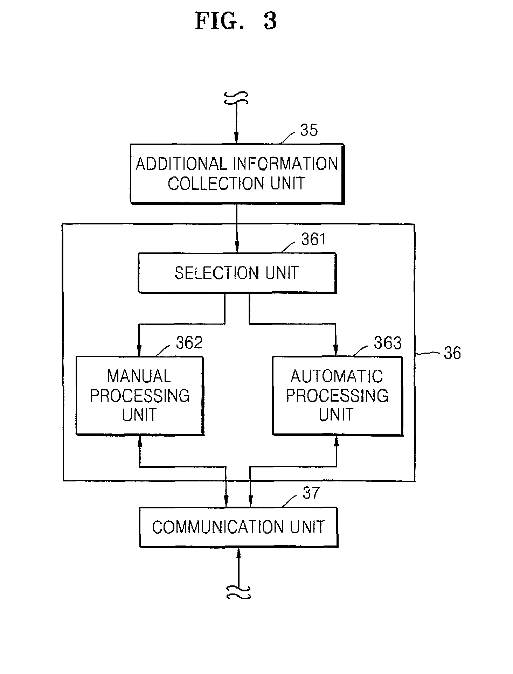

FIG. 3 is a detailed block diagram of an exemplary embodiment of the processing unit 36 of the patient management gateway 3 shown in FIG. 1. Referring to FIG. 3, the processing unit 36 comprises a selection unit 361, a manual processing unit 362, and an automatic processing unit 363.

The selection unit 361 selects any one of the medical processing methods based on the additional information collected by the additional information collection unit 35. The selection unit 361 may select any one of the medical processing methods based on the information initially collected by the information collection unit 33 in addition to the additional information collected by the additional information collection unit 35. In more detail, the selection unit 361 selects any one of the manual processing method and the automatic processing method according to whether the additional information collected by the additional information collection unit 35 requires the medical personnel to make any decisions to diagnose and treat a disease of the patient.

If the selection unit 361 selects the manual processing method, the manual processing unit 362 manually performs medical processing with regard to the patient according to instructions given by the medical personnel who recognizes a patient's condition that relates to the information collected by the information collection unit 33 and the additional information collected by the additional information collection unit 35. In more detail, the manual processing unit 362 analyzes the information collected by the information collection unit 33 and the additional information collected by the additional information collection unit 35, and prepares a report regarding the patient's condition based on the analysis result. Thereafter, the manual processing unit 362 notifies the medical personnel who handles the patient management server 1 of the patient's condition by transmitting the report to the patient management server 1 through the communication unit 37. In this regard, the manual processing unit 362 receives instructions from the medical personnel who receives the report regarding the patient's condition from the patient management server 1 through the communication unit 37, and performs medical processing with regard to the patient according to the received instructions. An example of the manual processing method according to the medical personnel's instructions is that the manual processing unit 362 transmits a prescription, an exercise plan, and a diet prepared by the medical personnel to the patient terminal 2 through the communication unit 37 so that the patient receives feedback from the medical personnel regarding disease management of the patient.

If the information collected by the information collection unit 33 does not correspond to the event indicating an abnormal condition of the patient or the selection unit 361 selects the automatic processing method as a result of monitoring by the event monitor 34, the automatic processing unit 363 automatically performs medical processing with regard to the patient based on the information collected by the information collection unit 33 and the additional information collected by the additional information collection unit 35. In another exemplary embodiment, the automatic processing unit 363 automatically performs medical processing with regard to the patient based on only the information collected by the information collection unit 33. In more detail, the automatic processing unit 363 analyzes the information collected by the information collection unit 33 and the additional information collected by the additional information collection unit 35, and prepares a report regarding the patient's condition based on the analysis result. Thereafter, the automatic processing unit 363 transmits the report to the patient terminal 2 through the communication unit 37 so that the patient automatically receives feedback regarding disease management.

Also, the automatic processing unit 363 may automatically perform medical processing with regard to the patient using a clinical decision support system ("CDSS") for automatically making a decision regarding medical processing with regard to the patient based on the information collected by the information collection unit 33 and the additional information collected by the additional information collection unit 35. In this regard, the CDSS has been proposed by Dr. Robert Hayward and assists the medical personnel with a decision making task regarding diagnosis and treatment. At present, there are about 10,000 CDSSs including a deafness diagnostic system, an allergic coryza diagnostic system, a bronchial asthma diagnostic system, a leukemia diagnostic system, a hypertension management system, etc.

In more detail, the automatic processing unit 363 provides the CDSS with the information initially collected by the information collection unit 33 and the additional information collected by the additional information collection unit 35. Thereafter, the CDSS analyzes the information initially collected by the information collection unit 33 and the additional information collected by the additional information collection unit 35, and makes a decision regarding medical processing according to the patient's condition based on the analysis result.
Thereafter, the automatic processing unit 363 automatically performs medical processing with regard to the patient according to the decision made by the CDSS. An example of the automatic medical processing according to the decision made by the CDSS may be transmission of the prescription, the exercise plan, and the diet prepared by the CDSS to the patient terminal 2 through the communication unit 37.

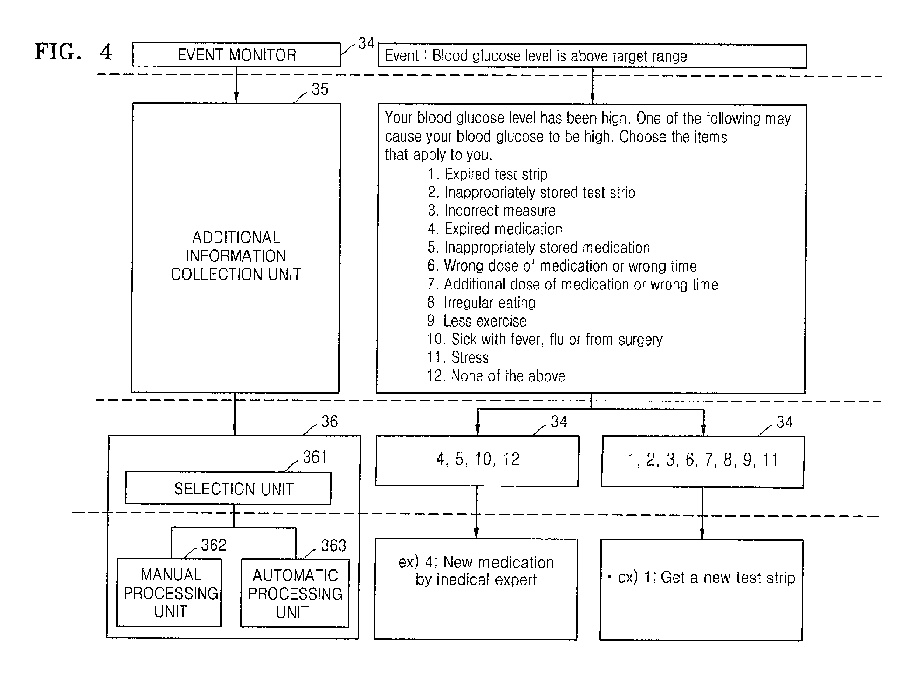

FIG. 4 illustrates a patient's blood glucose level applied to the patient management system shown in FIG. 1.

If information about the patient's blood glucose level collected by the information collection unit 33 exceeds the range indicating the patient's normal condition, the event monitor 34 monitors whether the patient's blood glucose level corresponds to any one of events included in the list stored in the medical information database 31. If the patient's blood glucose level exceeds the range indicating the patient's normal condition and is included in the events included in the list, the information collected by the information collection unit 33 corresponds to the event indicating the patient's abnormal condition.

If the information collected by the information collection unit 33 corresponds to an event indicating an abnormal condition of the patient as a result of monitoring obtained by the event monitor 34, the additional information collection unit 35 transmits a list of 12 pieces of additional information shown in FIG. 4 to the patient terminal 2 through the communication unit 37. If the patient selects at least one of the 12 pieces of additional information, the additional information collection unit 35 obtains additional information about a patient corresponding to the selected information. For example, if the patient selects a first item indicating that a test strip has expired, the additional information collection unit 35 obtains additional information indicating that the test strip of the patient has expired.

If the patient selects 4^{th}, 5^{th}, 10^{th}, and 12^{th} items from the 12 pieces of additional information, since the medical personnel makes a decision to diagnose and treat the patient, the selection unit 361 selects the manual processing method. For example, if the patient selects the 4^{th} item indicating a medicine prescription has expired, the selection unit 361 selects the manual processing method so that the medical personnel can write a new medicine prescription. If the patient selects 1^{st} through 3^{rd} and 6^{th} through 11^{th} items from the 12 pieces of additional information, since the medical personnel do not need to make a decision to diagnose and treat the patient, the selection unit 361 selects the automatic processing method. For example, if the patient selects the 1^{st} item indicating that the test strip of the patient has expired from the 12 pieces of additional information, the selection unit 361 selects the automatic processing method in order to automatically transfer a message instructing the patient to exchange test strips without the aid of the medical personnel.

If the patient selects the 4^{th} item indicating that the medicine prescription has expired from the 12 pieces of additional information, the manual processing unit 362 prepares a report informing the medical personnel that the medicine prescription of the patient has expired and transmits the report to the patient management server 1 through the communication unit 37 so that the medical personnel can write a new medicine prescription. Thereafter, the manual processing unit 362 transmits the new medicine prescription prepared by the medical personnel to the patient terminal 2 through the communication unit 37.

If the patient selects the 1^{st} item indicating that the test strip has expired from the 12 pieces of additional information, the automatic processing unit 363 prepares a report informing the patient to exchange the test strip for another test strip and transmits the report to the patient terminal 2 through the communication unit 37 in order to automatically transfer the message instructing the patient to exchange test strips without the aid of the medical personnel.

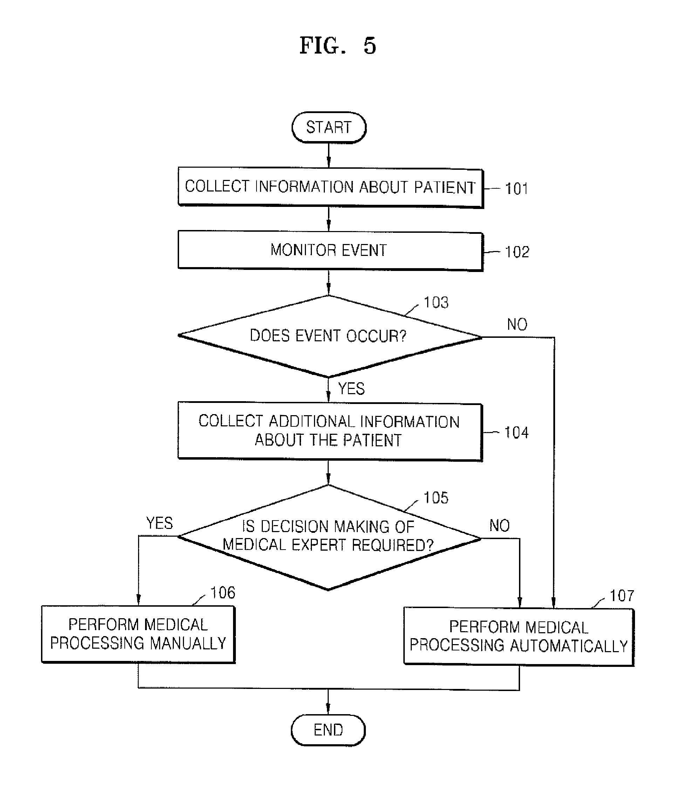

FIG. 5 is a flowchart illustrating an exemplary embodiment of a patient management method according to the inventive concept. Referring to FIG 5, the present exemplary embodiment of a patient management method includes operations sequentially processed by the patient management gateway 3 shown in FIG. 1. Therefore, although not described, the description with regard to the patient management gateway 3 is applied to the patient management method of the present exemplary embodiment.

In operation 101, the patient management gateway 3 collects information about a patient who uses the patient terminal 2, from the patient terminal 2. In operation 102, the patient management gateway 3 monitors if the information collected in operation 101 corresponds to an event indicating an abnormal condition of a patient, based on data stored in the medical information database 31 and the patient information database 32. In operation 103, as a result of the monitoring performed in operation 102, if the information collected in operation 101 corresponds to the event indicating the patient's abnormal condition, the patient management gateway 3 proceeds to operation 104, and, if the information collected in operation 101 does not correspond to the event indicating the patient's abnormal condition, the patient management gateway 3 proceeds to operation 107.

In operation 104, the patient management gateway 3 collects additional information about the patient. In operation 105, the patient management gateway 3 selects a medical processing method with regard to the patient according to whether the additional information collected in operation 104 requires medical personnel to make a decision to diagnose and treat a patient's disease. If the patient management gateway 3 selects a manual processing method, the patient management gateway 3 proceeds to operation 106, and if the patient management gateway 3 selects an automatic processing method, the patient management gateway 3 proceeds to operation 107. In operation 106, the patient management gateway 3 manually performs medical processing with regard to the patient according to instructions given by the medical personnel who recognizes a patient's condition indicated by the information obtained in operation 101 and the additional information obtained in operation 104. In operation 107, the patient management gateway 3 automatically performs medical processing with regard to the patient based on the information obtained in operation 101 and the additional information obtained in operation 104.

As described above, according to one or more of the above exemplary embodiments, detailed information about a patient is collected when a specific event occurs while basic information about the patient is collected normally, thereby removing patient inconvenience caused by a lot of unnecessary information gathering and reducing a load on a patient management system. In particular, when a patient's condition is good or a patient's disease is not serious, the patient management system automatically performs medical processing with regard to the patient without the aid of the medical personnel, thereby reducing the workload on the medical personnel.

If the patient's condition is good or the patient's disease is not serious, medical processing with regard to the patient is mostly automatically performed. Thus, disease management of the patient is more quickly fed back, medical expense is reduced, and a possibility of errors made by a person, i.e., human error, such as might be caused by the medical personnel, is reduced, thereby more efficiently managing the patient's disease.

In addition, other exemplary embodiments can also be implemented through computer readable code/instructions in/on a medium, e.g., a computer readable medium, to control at least one processing element to implement any above described embodiment. The medium can correspond to any medium/media permitting the storage and/or transmission of the computer readable code.

The computer readable code can be recorded/transferred on a medium in a variety of ways, with examples of the medium including recording media, such as magnetic storage media, exemplary embodiments of which include random access memory ("ROM"), floppy disks, hard disks, and other similar devices, and optical recording media, exemplary embodiments of which include compact disc ROMs ("CD-ROMs"), or digital video disks ("DVDs"), and transmission media such as media carrying or including carrier waves, as well as elements of the Internet.

It should be understood that the exemplary embodiments described herein should be considered in a descriptive sense only and not for purposes of limitation. Descriptions of features or aspects within each embodiment should typically be considered as available for other similar features or aspects in other embodiments.

## Claims

1. A patient management apparatus comprising:
a first collection unit which collects initial information about a patient;
a monitor which determines whether the collected initial information corresponds to an event indicating an abnormal condition of the patient;
a second collection unit which selectively collects additional information about the patient based on a result of the monitoring; and
a processing unit which performs medical processing with regard to the patient based on at least one of the collected initial information and the additional information.

2. The apparatus of claim 1, wherein the processing unit selects a medical processing method with regard to the patient based on the additional information and performs medical processing with regard to the patient according to the selected medical processing method.

3. The apparatus of claim 1 or 2, wherein the processing unit comprises:
a selection unit which selects a medical processing method with regard to the patient based on the additional information;
a manual processing unit which manually performs medical processing with regard to the patient according to medical personnel when a first medical processing method is selected; and
an automatic processing unit which automatically performs medical processing with regard to the patient when a second medical processing method is selected.

4. The apparatus of claim 3, wherein the manual processing unit sends a report regarding a condition of the patient to the medical personnel and performs medical processing with regard to the patient according to instructions given by the medical personnel who receives the report.

5. The apparatus of claim 3 or 4, wherein the automatic processing unit prepares a report regarding a condition of the patient and transmits the report to a patient terminal.

6. The apparatus of claim 3, 4 or 5, wherein the automatic processing unit uses a clinical decision support system to automatically perform medical processing with regard to the patient.

7. The apparatus of any one of the preceding claims, wherein the processing unit automatically performs medical processing with regard to the patient if the collected initial information does not correspond to the event indicating the abnormal condition of the patient.

8. A patient management method comprising:
collecting initial information about a patient;
monitoring the collected initial information to determine whether the collected initial information corresponds to an event indicating an abnormal condition of the patient;
selectively collecting additional information about the patient based on a result of the monitoring; and
performing medical processing with regard to the patient based on at least one of the collected initial information and the additional information.

9. The method of claim 8, wherein the performing of the medical processing comprises:
selecting a medical processing method with regard to the patient based on the additional information; and
performing medical processing with regard to the patient according to the selected medical processing method.

10. The method of claim 8 or 9, wherein the performing of the medical processing comprises:
selecting a medical processing method with regard to the patient based on the additional information;
manually performing medical processing with regard to the patient according to a medical personnel when a first medical processing method is selected; and
automatically performing medical processing with regard to the patient when a second medical processing method is selected.

11. The method of claim 10, wherein the manual performing of the medical processing comprises:
sending a report regarding a condition of the patient to the medical personnel; and
performing medical processing with regard to the patient according to instructions given by the medical personnel who receives the report.

12. The method of claim 10 or 11, wherein the automatic performing of the medical processing comprises:
preparing a report regarding the condition of the patient; and
transmitting the report to a patient terminal.

13. The method of any one of the preceding claims 8-12, wherein the performing of the medical processing comprises:
automatically performing medical processing with regard to the patient when the collected initial information does not correspond to the event indicating the abnormal condition of the patient.

14. The method of any of the preceding claims 8-13, wherein the performing of the medical processing comprises
consulting medical personnel based on at least one of the collected initial information and the additional information;
providing the medical personnel with detailed information in order to treat the patient; and
treating the patient directly, wherein the treating is performed by at least one of the medical personnel and an automated system.

15. A computer readable recording medium storing a program for executing a patient management method, the method comprising:
collecting initial information about a patient;
monitoring the collected initial information to determine whether the collected information corresponds to an event indicating an abnormal condition of the patient;
selectively collecting additional information about the patient based on a result of the monitoring; and
performing medical processing with regard to the patient based on at least one of the collected initial information and the additional information.
